# EUROPEAN PATENT APPLICATION

(11) **EP 4 570 180 A1**
(43) Date of publication of application: **18.06.2025**
(21) Application number: 24218497.6
(22) Date of filing: 09.12.2024
(51) Int. Cl.: A61B 6/03, A61B 6/00

(54) **NUCLEAR MEDICINE DIAGNOSIS DEVICE, REGION-OF-INTEREST MOVEMENT STATE DETECTION METHOD, AND PROGRAM**

(30) Priority: 13.12.2023 JP 2023209853
(71) Applicant: Canon Medical Systems Corporation, Tochigi 324-0036 (JP)
(72) Inventor: ONO, Shogo, Otawara-shi, 324-0036 (JP)
(74) Representative: Marks & Clerk LLP

(57) **Abstract**

According to an embodiment, a nuclear medicine diagnosis device includes a collector, a divider, and a detector. The collector collects radiation data obtained by detecting radiation based on radioactive medicine administered to a subject. The divider divides the radiation data into a plurality of radiation data segments with predetermined time widths. The detector detects the movement state of a region of interest in a body of the subject based on the radiation data segments.

## Description

### BACKGROUND OF THE INVENTION

### Field of the Invention

The present invention relates to a nuclear medicine diagnosis device, a region-of-interest movement state detection method, and a program.

### Description of Related Art

Conventionally, technologies related to systems and devices for a positron emission tomography (PET) examination have been disclosed. In the PET examination, a medical image (a PET image) is generated by administering radioactive medicine to a subject (a patient) and collecting radiation emitted by the administered radioactive medicine for a predetermined time.

Meanwhile, a portion in the patient's body where radioactive medicine is accumulated (an accumulation portion) is not limited to a portion where a tumor is located and a state in which the accumulation portion moves due to respiration or the like in a diagnosis process based on a PET image may be obtained as useful additional information for the diagnosis process. For example, if the accumulation portion is the tumor, periodic movement such as a change in respiration often does not occur when adhesion between the tumor and the surrounding tissue is occurring and the amount of movement may be large when adhesion between the tumor and the surrounding tissue is not occurring. Therefore, when a movement state of the tumor and the tissues surrounding the tumor is confirmed, the confirmed movement state may be useful information for determining a surgical method according to the adhesion between the tumor and the surrounding tissue in surgery and determining the prognosis after surgery. For example, when the accumulation portion is moving over time, because the accumulation portion may be intestinal content such as food residues in the intestinal tract, the movement state of the accumulation portion is confirmed, such that the confirmed movement state may be useful information for determining whether the accumulation portion is specific (positive) or non-specific (false positive).

For this reason, in conventional PET examinations, for example, a device such as a respiratory synchronization device that outputs information indicating a respiration state is attached to a patient, or a mark (marker) is attached to the patient and photographed by an imaging device such as a camera to track the mark. Also, radiation from the administered radioactive medicine is collected while the patient's respiration state is being monitored and a moving image for visually confirming the movement state of the accumulation portion, a so-called cine image, is generated so that a determination process is performed. When confirming the changes over time at the accumulation portion, for example, after the patient's entire body is photographed, additional photography is also performed for the accumulation portion.

However, the attachment of a device such as a respiratory synchronization device to a patient to generate a cine image in the conventional PET examination may cause a physical burden on the patient. In the conventional PET examination, it is possible to generate a cine image by performing photography without attaching a device such as a respiratory synchronization device to the patient. However, in this case, for example, a doctor, a technician, or the like needs to perform a complicated process different from a normal process after photography. Moreover, the visual confirmation of the movement state of the accumulation portion using the cine image is a qualitative confirmation method. On the other hand, when additional photography is performed after the patient's entire body is photographed in the conventional PET examination, the patient's exposure will increase because an additional CT imaging is needed. Furthermore, for example, if a doctor, a technician, or the like performs additional photography that is not planned, it may be a factor that affects the plan and schedule (schedule) of the PET examination.

### SUMMARY OF THE INVENTION

An objective of the present invention is to quantitatively detect a movement state of a region of interest during an examination to be performed by capturing a medical image of a subject. However, objectives of embodiments disclosed in the present specification and the drawings are not limited to the above-described objective. Objectives corresponding to effects of configurations shown in the embodiments to be described below can be considered as other objectives.

According to an embodiment, a nuclear medicine diagnosis device includes a collector, a divider, and a detector. The collector collects radiation data obtained by detecting radiation based on radioactive medicine administered to a subject. The divider divides the radiation data into a plurality of radiation data segments with predetermined time widths. The detector detects the movement state of a region of interest in a body of the subject based on the radiation data segments.

The detector may include a reconstructed image generator configured to generate reconstructed images indicating positions of the region of interest based on the radiation data segments; a rearrangementer configured to rearrange the generated reconstructed images in order of time of respiration of the subject; and a comparator configured to compare the positions of the region of interest between at least two rearranged reconstructed images, wherein the movement state of the region of interest may be detected based on a comparison result of performing a comparison process.

The rearrangementer may rearrange the reconstructed images based on feature quantities of the generated reconstructed images.

The rearrangementer may extract the feature quantities including at least movement components in the region of interest indicated in the reconstructed images by inputting the reconstructed images to a learning network and rearrange the reconstructed images based on a degree of similarity between the feature quantities.

The comparator may compare positions of the region of interest between a first reconstructed image that is the reconstructed image indicating a maximum expiration state in the respiration of the subject and a second reconstructed image that is the reconstructed image indicating a maximum inspiration state in the respiration of the subject.

The comparator may obtain a difference between the position of a first region of interest that is the region of interest indicated in the first reconstructed image and the position of a second region of interest that is the region of interest indicated in the second reconstructed image and output information indicating the obtained difference as the comparison result.

According to an embodiment, there is provided a movement state detection method including: collecting, by a computer, radiation data obtained by detecting radiation based on radioactive medicine administrated to a subject; dividing, by the computer, the radiation data into a plurality of radiation data segments with predetermined time widths; and detecting, by the computer, a movement state of a region of interest in a body of the subject based on the radiation data segments.

According to an embodiment, there is provided a program for causing a computer to: collect radiation data obtained by detecting radiation based on radioactive medicine administrated to a subject; divide the radiation data into a plurality of radiation data segments with predetermined time widths; and detect a movement state of a region of interest in a body of the subject based on the radiation data segments.

### BRIEF DESCRIPTION OF THE DRAWINGS

FIG. 1 is a configuration diagram of a nuclear medicine diagnosis device according to an embodiment.
FIG. 2 is a diagram showing an example of a functional configuration of a movement state detection function provided in the nuclear medicine diagnosis device according to the embodiment.
FIG. 3 is a flowchart showing an example of a series of processing steps performed in the movement state detection function provided in the nuclear medicine diagnosis device according to the embodiment.

### DETAILED DESCRIPTION OF THE INVENTION

Hereinafter, a nuclear medicine diagnosis device, a region-of-interest movement state detection method, and a program according to embodiments will be described with reference to the drawings. Nuclear medicine diagnosis devices include, for example, a medical diagnosis device, which acquires a medical image of a subject placed on a patient table device and performs a diagnosis process, such as a positron emission tomography (PET) device or a PET-computed tomography (CT) device. In the following description, a case where the nuclear medicine diagnosis device is a PET device will be described with reference to an example.

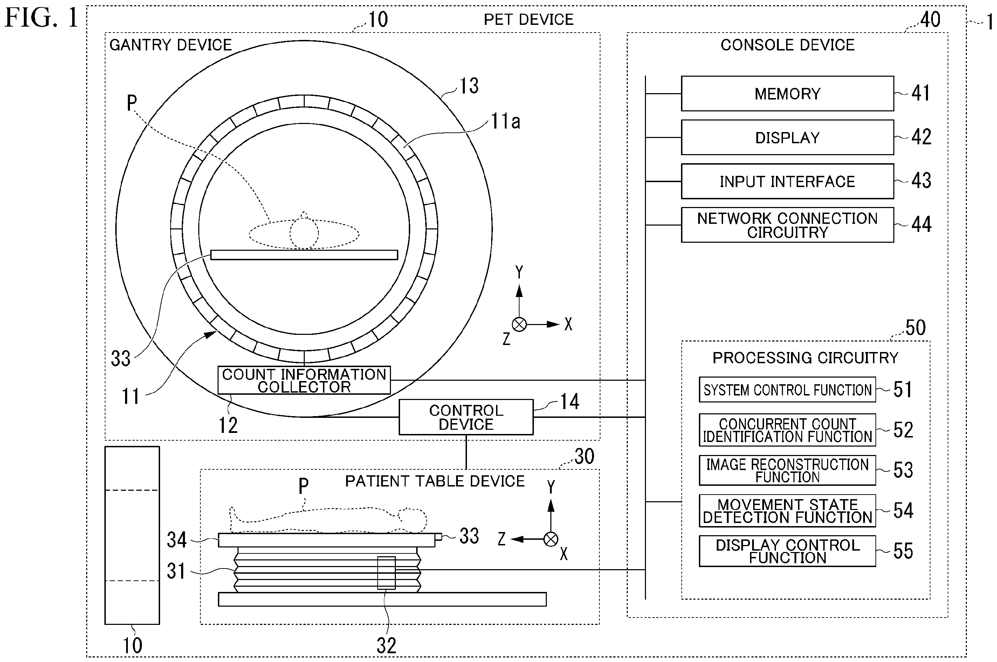

FIG. 1 is a configuration diagram of a nuclear medicine diagnosis device (a PET device) according to the embodiment. The PET device 1 is a medical diagnosis device that detects radiation emitted by a radioactive substance contained in radioactive medicine administered to a subject P (a patient), and determines the tendency of the radioactive medicine to accumulate from the amount of the detected radiation. In the PET device 1, an image corresponding to the amount of the detected radiation is generated and displayed. Thereby, a PET examination practitioner (a doctor, a technician, or the like) can visually confirm whether or not there is a lesion in the subject.

The PET device 1 includes, for example, a gantry device 10, a patient table device 30, and a console device 40. In FIG. 1, for the convenience of description, both a view of the gantry device 10 in the Z-axis direction and a view of the gantry device 10 in the X-axis direction are shown. However, in reality, the number of gantry devices 10 provided in the PET device 1 is one. In the present embodiment, a central axis of a frame 13 in a non-tilt state or a longitudinal direction of a top plate 33 of the patient table device 30 is defined as a Z-axis direction, an axis orthogonal to the Z-axis direction and horizontal to a floor surface is defined as an X-axis direction, and a direction orthogonal to the Z-axis direction and perpendicular to the floor surface is defined as a Y-axis direction.

The PET device 1 is an example of a "nuclear medicine diagnosis device."

The gantry device 10 includes, for example, a radiation detector 11, a count information collector 12, a frame 13, and a control device 14.

The radiation detector 11 detects radiation emitted from the subject P (more specifically, radioactive medicine administered to the subject P), for example, gamma rays. The radiation detector 11 outputs an electrical signal (which may be an optical signal or the like) corresponding to the amount of the detected radiation (a radiation amount) to the count information collector 12. The radiation detector 11 is formed in a cylindrical shape and is arranged to surround an imaging port formed in the gantry device 10. In the radiation detector 11, for example, a plurality of PET detection elements 11a are arranged in a circumferential direction and a central-axis direction. Each PET detection element 11a detects radiation emitted from the subject P located at the imaging port to the surroundings. The PET detection element 11a is, for example, a detector having a scintillator array and an optical sensor array. The scintillator array has a plurality of scintillators. Each scintillator causes scintillation in accordance with incident gamma rays and emits an amount of light corresponding to an intensity of the gamma rays. The optical sensor array includes, for example, an optical sensor such as a silicon-photomultiplier (SiPM) or a photomultiplier (PMT). The optical sensor array outputs an electrical signal (a pulse) with a wave height corresponding to the amount of light emitted by the scintillator. Thereby, the radiation detector 11 outputs a pulse signal corresponding to the radiation amount detected by each PET detection element 11a to the count information collector 12.

The count information collector 12 generates count information corresponding to collected pulse signals by collecting the pulse signals output by each PET detection element 11a provided in the radiation detector 11 and assigning information about a position (Position: P), energy (Energy: E), and time (Timing: T) to an input event of radiation detected by each PET detection element 11a (hereinafter simply referred to as an "event"). The count information collector 12 includes, for example, processing circuitry having a processor such as a central processing unit (CPU) and executes functions that are a position information identification function, an energy identification function, and a timing identification function. The position information identification function is a function of identifying a gamma-ray emission position (a scintillation position) in the scintillator. When scintillator-specific pixels and silicon photomultiplier tubes are coupled on a one-to-one basis, the position of the scintillator-specific pixel corresponding to the silicon photomultiplier tube that outputs the pulse signal is identified in the position information identification function. When a small number of photomultiplier tubes are associated with a large number of scintillator-specific pixels, a light emission position in the scintillator is estimated by calculating the center of gravity of the pulse signals output by the plurality of silicon photomultiplier tubes in the position information identification function. The energy identification function is a function of estimating the energy of the pulse signal output by the optical sensor. In the energy identification function, for example, energy is estimated to be high in the case of high energy of 511 keV gamma rays or the like and estimated to be low in the case of low energy of a scattering event or the like. As an energy estimation method in the energy identification function, for example, there is an energy integration method of converting a range in which a pulse signal exceeds a predetermined threshold value into energy by performing an energy integration process, a time-over-threshold (ToT) method of converting a time width in which the pulse signal exceeds a predetermined threshold value into energy, or the like. In the estimation of energy based on the ToT method, the nonlinearity of an estimation result is also corrected. The timing identification function is a function of identifying a timing at which the pulse signal output by the optical sensor exceeds a predetermined threshold value as timing information. The count information collector 12 outputs data of the count information (hereinafter referred to as "single list mode data") generated by executing these functions to the console device 40.

The frame 13 is an annular member that supports the radiation detector 11 and the count information collector 12. The frame 13 may be a member such as an arm as long as it can support the radiation detector 11 and the count information collector 12 and is not limited to the annular member.

When the nuclear medicine diagnosis device of the present embodiment is, for example, a PET-CT device, the frame 13 may rotatably support a rotary frame that fixes an X-ray tube configured to generate X-rays to be applied to the subject P and an X-ray detector configured to detect an intensity of the X-rays incident through the subject P at opposite positions as well as the radiation detector 11 and the count information collector 12 in a state in which the subject P introduced inside thereof is defined as the center.

The control device 14 receives an input signal from an input interface (not shown) such as a manipulation switch attached to the gantry device 10 or an input interface 43 attached to the console device 40 and controls operations of the gantry device 10 and the patient table device 30. The control device 14 includes, for example, processing circuitry having a processor such as a CPU, and a drive mechanism including a motor, an actuator, or the like that moves the gantry device 10 or moves the top plate 33 of the patient table device 30. Although a case where the control device 14 is provided in the gantry device 10 is shown in the embodiment, the control device 14 may be provided in the console device 40. As used herein, the input interface is not limited to an input interface including physical manipulation elements such as a mouse and a keyboard. For example, examples of the input interface include electrical signal processing circuitry that receives an electrical signal corresponding to an input manipulation from an external input device provided separately from the device and outputs this electrical signal to a control circuit.

The control device 14, for example, may tilt the gantry device 10, horizontally move a housing of the gantry device 10 (hereinafter simply referred to as "the gantry device 10") in the longitudinal direction (the Z-axis direction) of the top plate 33 of the patient table device 30, or vertically move the top plate 33 of the patient table device 30 in the Y-axis direction (wherein the movement may include lateral movement in the X-axis direction and rotational movement around the Z-axis). When the gantry device 10 is tilted, the control device 14 tilts the frame 13 around an axis parallel to the Z-axis direction on the basis of a tilt angle input to the input interface (not shown) or the input interface 43. The control device 14 ascertains the tilt angle of the frame 13 according to an output of a sensor (not shown) or the like. The control device 14 provides the tilt angle of the frame 13 to processing circuitry 50 at any time.

When the nuclear medicine diagnosis device of the present embodiment is, for example, a PET-CT device, the control device 14 may include a drive mechanism including, for example, a motor or an actuator that rotates the rotary frame provided in the frame 13.

The patient table device 30 is a device for placing and moving the subject P to be scanned and introducing the subject P into the frame 13 of the gantry device 10. The patient table device 30 includes, for example, a base 31, a patient-table drive device 32, a top plate 33, and a support frame 34. The base 31 includes a housing that can movably support the support frame 34 in a perpendicular direction (the vertical direction or the Y-axis direction). The patient-table drive device 32 includes a motor or an actuator. The patient-table drive device 32 moves the top plate 33 on which the subject P is placed along the support frame 34 in the longitudinal direction (the Z-axis direction) of the top plate 33. The amount of movement for causing the patient-table drive device 32 to move the top plate 33 in the longitudinal direction is the amount of movement for introducing a part of the subject P that has not been introduced into the frame 13 into the frame 13, i.e., compensating for the amount of horizontal movement of the gantry device 10, even if the control device 14 horizontally moves the gantry device 10 to the maximum. The patient-table drive device 32 may laterally move the top plate 33 on which the subject P is placed in the horizontal direction (the X-axis direction) or rotatably move the top plate 33 around the Z-axis. The patient-table drive device 32 may have a configuration in which both the gantry device 10 and the top plate 33 are moved. The patient-table drive device 32 may move the top plate 33 and the support frame 34 in the longitudinal direction of the top plate 33. The top plate 33 is a plate-shaped member on which the subject P is placed.

The console device 40 includes, for example, a memory 41, a display 42, the input interface 43, network connection circuitry 44, and the processing circuitry 50. Although a case where the console device 40 is separate from the gantry device 10 has been described in the present embodiment, the gantry device 10 may include some or all of the constituent elements of the console device 40.

The memory 41 is implemented by, for example, a semiconductor memory element such as a read-only memory (ROM), a random-access memory (RAM), or a flash memory, a hard disk drive (HDD), an optical disc, or the like. The memory 41 stores data of each processing step in the PET device 1. The memory 41 stores, for example, single list mode data output by the count information collector 12, a PET image created on the basis of the single list mode data, and data such as list mode data segments and reconstructed images to be described below created on the basis of the single list mode data. These data items may be stored in an external memory with which the PET device 1 can communicate in place of the memory 41 (or in addition to the memory 41). An external memory is controlled by a cloud server, for example, when the cloud server that manages the external memory receives a read/write request. The external memory may be implemented, for example, by a system referred to as a picture archiving and communication system (PACS). The PACS is a medical image management system that systematically stores images captured by various types of image diagnosis devices.

The display 42 displays various types of information. For example, the display 42 displays an image such as a PET image generated by the processing circuitry 50, a graphical user interface (GUI) image for receiving various types of manipulations from a PET examination practitioner, and the like. The display 42 is, for example, a liquid crystal display (LCD), a cathode ray tube (CRT) display, an organic electroluminescence (EL) display, or the like. The display 42 may be provided in the gantry device 10. The display 42 may be of a desktop type or may be a display device (for example, a tablet terminal) capable of wireless communication with a main body of the console device 40.

The input interface 43 receives various types of input manipulations from the PET examination practitioner and outputs an electrical signal indicating content of the received input manipulation to the processing circuitry 50. For example, the input interface 43 receives input manipulations for collection conditions for collecting single list mode data, reconstruction conditions for reconstructing PET images, and the like. The input interface 43 is implemented by, for example, a mouse, a keyboard, a touch panel, a trackball, a switch, a button, a joystick, a camera, an infrared sensor, a microphone, and the like. The input interface 43 may be provided as a manipulation switch with a function of receiving some input manipulations (in particular, a function of horizontally moving the housing of the gantry device 10) or the like in the gantry device 10. The input interface 43 may be implemented by a display device (for example, a tablet terminal) capable of wireless communication with a main body portion of the console device 40. As used herein, the input interface 43 is not limited to an input interface including physical manipulation elements such as a mouse and a keyboard. For example, examples of the input interface include electrical signal processing circuitry configured to receive an electrical signal corresponding to the input manipulation from an external input device provided separately from the device and output this electrical signal to the control circuit.

The network connection circuitry 44 includes, for example, a network card having a printed circuit board, a wireless communication module, and the like. The network connection circuitry 44 implements an information communication protocol corresponding to a form of the network to be connected. The network includes, for example, a local area network (LAN), a wide area network (WAN), the Internet, a cellular network, a leased line, and the like. The network connection circuitry 44, for example, implements a connection between an external memory implemented by the above-described PACS or the like and the console device 40.

The processing circuitry 50 controls the overall operation of the PET device 1. The processing circuitry 50 executes, for example, a system control function 51, a concurrent count identification function 52, an image reconstruction function 53, a movement state detection function 54, a display control function 55, and the like. The processing circuitry 50 implements these functions by, for example, executing a program (software) stored in the memory 41 with a hardware processor.

The hardware processor is, for example, circuitry such as a CPU, a graphics processing unit (GPU), an application-specific integrated circuit (ASIC), or a programmable logic device (for example, a simple programmable logic device (SPLD), a complex programmable logic device (CPLD), or a field programmable gate array (FPGA)). Instead of storing the program in the memory 41, the program may be directly embedded in the circuitry of the hardware processor. In this case, the hardware processor implements each function by reading and executing the program embedded in the circuitry. The hardware processor is not limited to being configured as a single circuit and may be configured as one hardware processor by combining a plurality of independent circuits to implement each function. A plurality of constituent elements may be integrated into one hardware processor to implement each function. Each function may be implemented by incorporating a plurality of constituent elements into one dedicated LSI circuit. Here, the program (software) may be stored in advance in a storage device (a storage device having a non-transitory storage medium) that constitutes a semiconductor memory device such as a ROM, a RAM, or a flash memory or a storage device such as a hard disk drive. Alternatively, the program (software) may be stored in a removable storage medium (a non-transitory storage medium) such as a DVD or a CD-ROM and installed in a storage device provided in the console device 40 when the storage medium is mounted in a drive device provided in the console device 40. The program (software) may be downloaded in advance from another computer device via the network to which the network connection circuitry 44 is connected and installed in the storage device provided in the console device 40. A program (software) installed in the storage device provided in the console device 40 may be transferred to and executed by processing circuitry provided in the control device 14.

Constituent elements of the console device 40 or the processing circuitry 50 may be distributed and implemented by a plurality of hardware elements. The processing circuitry 50 may be implemented by a processing device capable of communicating with the console device 40 instead of a configuration in which the console device 40 is provided. The processing device, for example, is a device (for example, a cloud server) connected to a workstation connected to one PET device, or a plurality of PET devices and configured to collectively execute processes equivalent to those of the processing circuitry 50 to be described below. That is, the configuration of the present embodiment can be implemented as a PET examination system (a nuclear medicine diagnosis system) in which a PET device and another processing device are connected via a network.

The system control function 51 is performed to control various types of functions of the processing circuitry 50 on the basis of, for example, an input manipulation received by the input interface 43. For example, the system control function 51 is performed to control a single list mode data collection process or the like in the gantry device 10 by controlling the count information collector 12, the control device 14, and the patient-table drive device 32.

The concurrent count identification function 52 is performed to generate list mode data by performing a concurrent count (coincidence) process for events included in the single list mode data output by the count information collector 12. When the list mode data is generated, the concurrent count identification function 52 is performed to pair events detected within a time window of a predetermined time width (e.g., a time width in units of p [sec]) so that two events detected at substantially the same time are identified. The function of the concurrent count identification function 52 may be performed by the count information collector 12. The concurrent count identification function 52 is performed to output the generated list mode data to the image reconstruction function 53. The concurrent count identification function 52 may be performed to store the generated list mode data in the memory 41 and output a notification indicating that the generated list mode data is stored to the image reconstruction function 53. The concurrent count identification function 52 may be performed to output the generated list mode data to the movement state detection function 54.

The image reconstruction function 53 is performed to perform a predetermined reconstruction process for the list mode data output by the concurrent count identification function 52 to generate a PET image that is a medical image of the subject P. Examples of the PET image reconstruction method in the image reconstruction function 53 include a maximum likelihood-expectation maximization (ML-EM) method, an ordered subsets-expectation maximization (OS-EM) method, which accelerates the ML-EM method, and the like.

The movement state detection function 54 is performed to detect the movement state of the region of interest (ROI) in the PET examination on the basis of the single list mode data output by the count information collector 12. That is, the movement state detection function 54 is performed to detect a movement state of a portion (an accumulation portion) inside the body in which the radioactive medicine administered to the subject P is accumulated moved due to the respiration of the subject P or the like. The region of interest is, for example, an accumulation portion to be examined in the body of the subject P designated by the PET examination practitioner by performing various types of input manipulations and received by the input interface 43. As the region of interest, for example, an accumulation portion (a light emitting position) in the PET image generated by the image reconstruction function 53 and displayed on the display 42 may be designated by the PET examination practitioner. Thereby, the PET examination practitioner can confirm the state of adhesion between the region of interest and the surrounding tissue and the movement of the region of interest over time by confirming the movement state of the region of interest. The configuration of the movement state detection function 54 and the operation of detecting the movement state of the region of interest will be described below.

The display control function 55 is performed to control a display mode of the display 42. For example, the display control function 55 is performed to control the display 42 so that the display 42 displays a PET image generated by the processing circuitry 50 (more specifically, the image reconstruction function 53), a GUI image for receiving various types of manipulations from a manipulator (a doctor, a technician, or the like) of the PET device 1, and the like. The display control function 55 also causes the display 42 to display an information image for presenting the movement state of the region of interest detected by the movement state detection function 54 and the like to the PET examination practitioner.

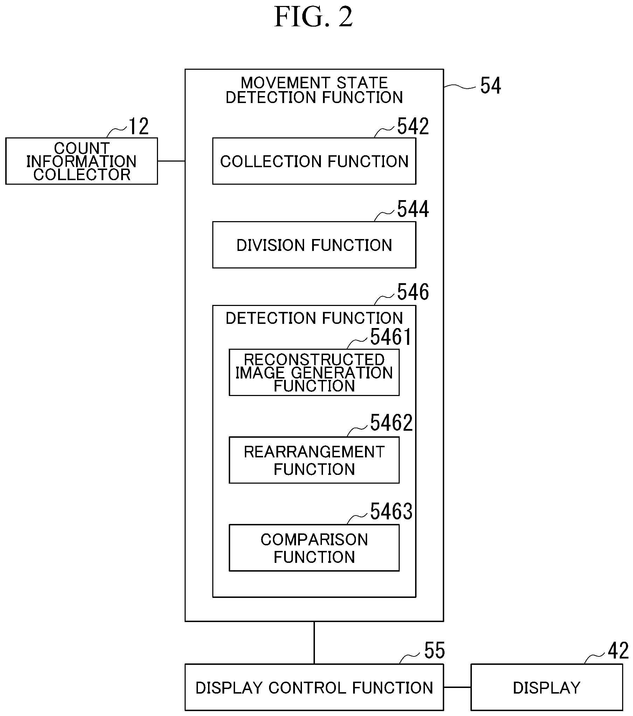

Next, a configuration and operation for implementing the movement state detection function 54 will be described. FIG. 2 is a diagram showing an example of a functional configuration of the movement state detection function 54 provided in the nuclear medicine diagnosis device (the PET device 1) according to the embodiment. In FIG. 2, the count information collector 12, the display control function 55, and the display 42 related to a process of detecting the movement state of the region of interest in the movement state detection function 54 are shown together. The movement state detection function 54 includes, for example, a collection function 542, a division function 544, and a detection function 546. The detection function 546 includes, for example, a reconstructed image generation function 5461, a rearrangement function 5462, and a comparison function 5463.

The collection function 542 is performed to collect a series of single list mode data items output by the count information collector 12. The collection function 542 may be performed to collect the single list mode data stored in the memory 41. Here, information about the position (P), energy (E), and time (T) of the event as described above is assigned to each single list mode data item. The collection function 542 is performed to output each collected single list mode data item to the division function 544. The collection function 542 may be performed to store the collected series of single list mode data items in the memory 41 and output a notification indicating that the collected series of single list mode data items is stored to the division function 544. The list mode data output by the concurrent count identification function 52 may be acquired.

The collection function 542 is an example of a "collector." The single list mode data (which may be list mode data) collected by the collection function 542 is an example of "radiation data."

The division function 544 is performed to divide a series of single list mode data items output by the collection function 542 into predetermined time widths (time division). More specifically, the division function 544 is performed to perform a classification process for each predetermined time width on the basis of the information about an event time (T) assigned to each single list mode data item. The division function 544 is performed to divide a series of single list mode data items output by the collection function 542 into at least two or more time widths (time division). The predetermined time width in which the division function 544 is performed to classify a series of single list mode data items is, for example, 1 [sec]. In this regard, the division function 544 is performed to perform a classification process so that time widths overlap. The overlapping time is, for example, 0.5 [sec]. Thereby, the series of single list mode data items output by the collection function 542, for example, is divided into a single list mode data group belonging to a time width of 0 to 1 [sec] and a single list mode data group belonging to a time width of 0.5 to 1.5 [sec] by the division function 544. The division function 544 is performed to output the single list mode data groups divided into predetermined time widths to the detection function 546. The collection function 542 may be performed to store the single list mode data groups in the memory 41 and output a notification indicating that the single list mode data groups are stored to the detection function 546. In the following description, the single list mode data groups divided by the division function 544 into the time widths are referred to as "list mode data segments."

The division function 544 is an example of a "divider." The list mode data segment (the single list mode data group) obtained in a division process of the division function 544 is an example of a "radiation data segment."

The detection function 546 is performed to detect the movement state of the region of interest by calculating the amount of movement of the region of interest (the accumulation portion) in the PET examination on the basis of the list mode data segment output by the division function 544.

The reconstructed image generation function 5461 is performed to generate a reconstructed image corresponding to the list mode data segment output by the division function 544. The reconstructed image generation function 5461 is performed to generate at least a reconstructed image indicating the position of the region of interest. As a method of generating the reconstructed image in the reconstructed image generation function 5461, for example, a technique for more accurately estimating a distance from a physical object (here, a gamma-ray emission position including the region of interest) such as a time of flight (TOF) can be used. In this case, the reconstructed image generation function 5461, for example, is performed to identify the position of the region of interest by measuring a time difference when two corresponding PET detection elements 11a provided in the radiation detector 11 detect radiation emitted in two directions from the portion of the region of interest. Here, the number of single list mode data items used by the reconstructed image generation function 5461 to identify the position of the region of interest is smaller than the number of single list mode data items used by the concurrent count identification function 52 to generate the list mode data used by the image reconstruction function 53, when a PET image is generated, but the reconstructed image generation function 5461 may be performed to generate a reconstructed image by performing a process similar to that to be performed by the concurrent count identification function 52 and the image reconstruction function 53. The reconstructed image generation function 5461 is performed to output the generated reconstructed image to the rearrangement function 5462. The reconstructed image generation function 5461 may be performed to cause the memory 41 to store the generated reconstructed image and output a notification indicating that the generated reconstructed image is stored to the rearrangement function 5462.

The reconstructed image generation function 5461 is an example of the "reconstructed image generator."

The rearrangement function 5462 is performed to rearrange the reconstructed image output by the reconstructed image generation function 5461 in order of time of respiration of the subject P. The rearrangement function 5462, for example, uses a function of a learning network based on artificial intelligence (Al) to extract a feature quantity including at least a component (a movement component) indicating a state in which the region of interest captured in each reconstructed image is moving. For example, a learning network sequentially extracts feature quantities associated with the respiration of the subject P from the input reconstructed image using a convolutional neural network (CNN) or a deep neural network (DNN). The feature quantity may include, for example, information indicating a respiration state in one cycle of respiration of the subject P or information indicating a time in one cycle of respiration of the subject P. The rearrangement function 5462 is performed to rearrange reconstructed images in order of time by arranging the reconstructed images at any time between the maximum expiration state and the maximum inspiration state within one cycle of respiration of the subject P on the basis of a degree of similarity between feature quantities extracted from the reconstructed images. The maximum expiration state is the most stable expiration phase defined by the PET examination practitioner. The maximum expiration state, for example, may be defined by the PET examination practitioner performing various types of input manipulations.

The rearrangement function 5462, for example, may be performed to rearrange the reconstructed images in order of time of the respiration of the subject P on the basis of the time (T) information of the event assigned to each single list mode data item belonging to the list mode data segment used to generate the reconstructed image. In this case, for example, the division function 544 may be performed to output the list mode data segment to which time information indicating each single list mode data item belonging to the list mode data segment is assigned to the detection function 546 and the reconstructed image generation function 5461 may be performed to assign the time information to the generated reconstructed image. The time information is, for example, information indicating the first, center, or last time width in the time width of the list mode data segment (the single list mode data group). The time information may be, for example, the time (T) information of the event assigned to the single list mode data representing the list mode data segment. Thereby, the rearrangement function 5462 can be performed to rearrange reconstructed images in order of time on the basis of the time information assigned to the reconstructed image.

In a PET examination, for example, when a device such as a respiratory synchronization device that outputs information indicating the state of respiration is attached to the subject P or a mark (marker) is attached to the subject P and photographed by an imaging device such as a camera to track the mark so that the respiration state of the subject P is monitored, the rearrangement function 5462 may be performed to rearrange the reconstructed images in order of time of the respiration of the subject P with reference to information indicating the state of respiration of the subject P output from the respiratory synchronization device or the like.

The rearrangement function 5462 is performed to assign information indicating the rearrangement order to each reconstructed image output by the reconstructed image generation function 5461 and output the assigned information to the comparison function 5463. The rearrangement function 5462 may be performed to assign information indicating the rearrangement order to the reconstructed image stored in the memory 41 and output a notification indicating that information indicating the order is assigned to the comparison function 5463. The rearrangement function 5462 may be performed to store each reconstructed image to which the information indicating the rearrangement order is assigned in the memory 41 and output a notification indicating that each reconstructed image is stored to the rearrangement function 5462. In the following description, when the reconstructed image generated by the reconstructed image generation function 5461 and the reconstructed image rearranged by the rearrangement function 5462 are distinguished, the reconstructed image rearranged by the rearrangement function 5462 is referred to as a "rearranged reconstructed image."

The rearrangement function 5462 is an example of a "rearrangementer."

The comparison function 5463 is performed to compare the reconstructed image indicating the maximum expiration state of the respiration of the subject P and the reconstructed image indicating the maximum inspiration state among the rearranged reconstructed images output by the rearrangement function 5462. More specifically, the comparison function 5463 is performed to compare the position of the region of interest captured in the reconstructed image indicating the maximum expiration state of the respiration of the subject P with the position of the region of interest captured in the reconstructed image indicating the maximum inspiration state. The comparison function 5463, for example, is performed to obtain a difference between a reconstructed image of a time width corresponding to the maximum expiration state (hereinafter referred to as an "expiration image") and a reconstructed image of a time width corresponding to the maximum inspiration state (hereinafter referred to as an "inspiration image") so that a quantitative comparison process for positions of the region of interest is performed. The comparison function 5463 is performed to compare the first expiration image with the inspiration image and then compare the expiration image with the next inspiration image to perform a comparison process for one cycle of respiration of the subject P. The comparison function 5463 is performed to output information indicating a difference between the positions of the region of interest obtained by comparing the expiration image and the inspiration image as a comparison result. At this time, the comparison function 5463 is performed to include information indicating a direction in which the position of the region of interest has changed, i.e., a direction of movement of the region of interest, in the comparison result and output the comparison result. More specifically, if the movement direction of the region of interest is a positive direction, for example, when the position of the region of interest becomes a position captured in the inspiration image from the position captured in an initial expiration image, the comparison function 5463 is performed to include information indicating whether the movement direction when the position of the region of interest becomes the position captured in the next expiration image from the position captured in the inspiration image is a positive direction or a negative direction opposite the positive direction in the comparison result and output the comparison result.

The comparison function 5463 is an example of a "comparator." The expiration image is an example of a "first reconstructed image" and the region of interest captured in the reconstructed image indicating the maximum expiration state of the respiration of the subject P is an example of a "first region of interest." The inspiration image is an example of a "second reconstructed image" and the region of interest captured in the reconstructed image indicating the maximum inspiration state of the respiration of the subject P is an example of a "second region of interest."

The detection function 546 is performed to detect the movement state of the region of interest on the basis of the information indicating the difference between the positions of the region of interest included in the comparison result output by the comparison function 5463 and the information indicating the movement direction of the region of interest. In other words, the detection function 546 is performed to detect a quantitative movement state including a movement amount and a movement direction indicating whether the position of the region of interest is changing to go back and forth synchronously with the respiration cycle of the subject P or changing over time in a similar direction regardless of the respiration cycle of the subject P. The detection function 546 is performed to output information indicating the movement state of the detected region of interest to the display control function 55 as the movement state of the region of interest detected by the movement state detection function 54. Thereby, the display control function 55 is performed to generate an information image indicating the movement state of the region of interest output by the detection function 546, output the information image to the display 42 so that the display 42 displays the information image, and present the information image to the PET examination practitioner.

The detection function 546 is performed to concatenate the rearranged reconstructed images output by the rearrangement function 5462 in order of time of the respiration of the subject P and further iterate the concatenation process at respiration cycles of the subject P so that a moving image for enabling a state in which the region of interest is moving in accordance with a respiration state of the subject P to be confirmed may be generated. That is, the detection function 546 may be performed to generate a moving image similar to a so-called cine image. The detection function 546 is performed to output the generated moving image to the display control function 55. The detection function 546 may be performed to output the rearranged reconstructed images output by the rearrangement function 5462 to the display control function 55 and the display control function 55 may be performed to generate a moving image similar to a cine image. The display control function 55 is performed to output a moving image output by the detection function 546 or a moving image generated by itself to the display 42 so that the display 42 displays the moving image. Thereby, the PET examination practitioner can confirm a state in which the region of interest is moving in accordance with the respiration state of the subject P.

The detection function 546 is an example of a "detector."

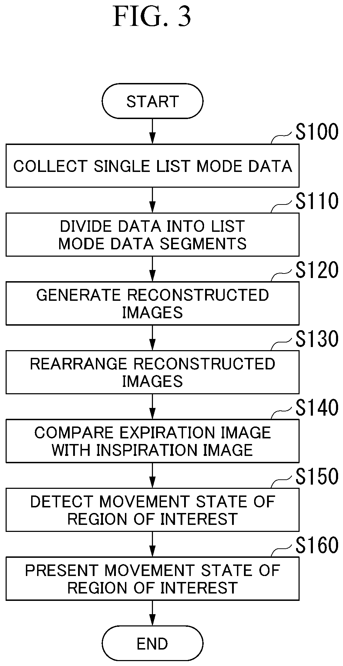

FIG. 3 is a flowchart showing an example of a series of processing steps performed by the movement state detection function 54 provided in the nuclear medicine diagnosis device (the PET device 1) according to the embodiment. When the manipulator of the PET device 1 manipulates the input interface 43 and starts photography based on the PET device 1, the movement state detection function 54 is performed to start a process of detecting the movement state of the region of interest in parallel with the process performed by the concurrent count identification function 52 and the image reconstruction function 53. In the following description, it is assumed that the region of interest is designated by the PET examination practitioner and the maximum expiration state is defined.

If the process of detecting the movement state of the region of interest is started in the movement state detection function 54, the collection function 542 is performed to collect single list mode data output by the count information collector 12 (step S100). The collection function 542 is performed to output each collected single list mode data item to the division function 544.

The division function 544 is performed to divide each single list mode data item output by the collection function 542 (time division) (step S110). The division function 544 is performed to output each list mode data segment to the detection function 546.

The reconstructed image generation function 5461 provided in the detection function 546 is performed to generate a reconstructed image corresponding to the list mode data segment output by the division function 544 (step S120). The reconstructed image generation function 5461 is performed to output the generated reconstructed image to the rearrangement function 5462.

The rearrangement function 5462 provided in the detection function 546 is performed to rearrange reconstructed images output by the reconstructed image generation function 5461 (step S130). More specifically, the rearrangement function 5462 is performed to sequentially extract feature quantities related to the respiration of the subject P by the function of the AI learning network and rearrange the reconstructed images in order of time of respiration of the subject P on the basis of a degree of similarity between the extracted feature quantities. The rearrangement function 5462 is performed to output the rearranged reconstructed images (the reconstructed images to which information indicating the rearrangement order is assigned) to the comparison function 5463.

The comparison function 5463 provided in the detection function 546 is performed to compare the inspiration image and the expiration image among the rearranged reconstructed images output by the rearrangement function 5462 (step S140). More specifically, the comparison function 5463 is performed to obtain a difference between the expiration image and the inspiration image so that a quantitative comparison process for positions of the region of interest for one cycle of respiration of the subject P is performed. The comparison function 5463 is performed to output information about a comparison result of comparing the expiration image with the inspiration image (the difference between the positions of the region of interest and the direction of movement of the region of interest).

The detection function 546 is performed to detect the movement state of the region of interest on the basis of the comparison result output by the comparison function 5463 (step S150). The detection function 546 is performed to output information indicating the detected movement state of the region of interest to the display control function 55. Thereby, the display control function 55 is performed to cause the display 42 to display an information image indicating the movement state of the region of interest output by the movement state detection function 54 (more specifically, the detection function 546) and present the information image to the PET examination practitioner (step S160).

According to the above-described configuration and process, in the PET device 1, the movement state detection function 54 is performed to collect the single list mode data and detect the movement state of the region of interest in the PET examination. More specifically, in the PET device 1, the collection function 542 is performed to collect the single list mode data and the division function 544 is performed to divide the single list mode data into predetermined time widths (time division), and the detection function 546 is performed to detect the movement state of the region of interest by calculating the amount of movement of the region of interest on the basis of each single list mode data group (list mode data segment). At this time, in the detection function 546, the reconstructed image generation function 5461 is performed to generate a reconstructed image corresponding to the list mode data segment, the rearrangement function 5462 is performed to rearrange generated reconstructed images in order of time of respiration of the subject P, and the comparison function 5463 is performed to quantitatively detect the movement state of the region of interest by obtaining a difference between the reconstructed image (expiration image) indicating the maximum expiration state and the reconstructed image (inspiration image) indicating the maximum inspiration image. Also, in the PET device 1, the display control function 55 is performed to present information about the movement state of the region of interest detected by the movement state detection function 54 to the PET examination practitioner. Thereby, the PET examination practitioner can confirm the movement state of the region of interest detected by the movement state detection function 54 and determine whether a state of adhesion between the region of interest and the surrounding tissue or the region of interest is specific (positive) or nonspecific (false positive).

Moreover, in the PET device 1, by using the single list mode data collected by the count information collector 12 during the PET examination, for example, it is possible to detect the movement state of the region of interest without attaching a device such as a respiratory synchronization device that outputs information indicating the state of respiration (a mark (marker) may be attached to the subject P and photographed with an imaging device such as a camera and the state of respiration may be obtained by tracking the mark) to the subject P. For this reason, the PET device 1 can detect the movement state of the region of interest without causing a physical burden on the subject P and without performing additional photography.

As described above, in the PET device 1, which is the nuclear medicine diagnosis device of the embodiment, the movement state detection function 54 is performed to collect single list mode data and generate reconstructed images corresponding to single list mode data groups (list mode data segments) divided into predetermined time widths (time division). In the PET device 1 of the embodiment, the movement state detection function 54 is performed to rearrange the generated reconstructed images in order of time of respiration of the subject P and detect a quantitative movement state of the region of interest by obtaining a difference between the reconstructed image (expiration image) indicating the maximum expiration state and the reconstructed image (inspiration image) indicating the maximum inspiration state. Thereby, in the PET device 1 of the embodiment, information about the movement state of the region of interest can be presented to the PET examination practitioner. In other words, in the PET device 1 of the embodiment, additional information useful for a diagnosis process based on the PET examination indicating a state in which the region of interest moves in accordance with the respiration of the subject P or the like can be presented to the PET examination practitioner. Thereby, the practitioner who performs the PET examination using the PET device 1 of the embodiment confirms the movement state of the region of interest and determines whether the state of adhesion between the region of interest and the surrounding tissues or the region of interest is specific (positive) or nonspecific (false positive).

Moreover, in the PET device 1 of the embodiment, in addition to a process of collecting single list mode data in the PET examination, for example, a device such as a respiratory synchronization device or a camera can detect the movement state of the region of interest without obtaining information indicating the respiration state of the subject P. That is, the PET device 1 of the embodiment can detect the movement state of the region of interest without causing a physical burden on the subject P as in the PET examination performed using a conventional PET device. Furthermore, in the PET device 1 of the embodiment, the movement state of the region of interest can be detected without additional imaging as in the case of a PET examination performed using a conventional PET device and the PET examination can be performed according to a prior plan or schedule.

Furthermore, in the PET device 1 of the embodiment, a moving image similar to a cine image generated using the conventional PET device can be generated by concatenating the reconstructed images based on the list mode data segments rearranged in order of time of respiration of the subject P. That is, in the PET device 1 of the embodiment, as in the case where a cine image is generated in the conventional PET device, it is possible to generate a moving image similar to a cine image in a simpler method without causing the PET examination practitioner to perform a complicated process different from a normal process after photography. Thereby, in the PET device 1 of the embodiment, the generated moving image can be presented to the PET examination practitioner. Thereby, the PET examination practitioner using the PET device 1 of the embodiment can visually confirm a state in which the region of interest is moving according to the respiration state of the subject P.

Although a configuration in which a function provided in the movement state detection function 54 is executed by the processing circuitry 50 provided in the PET device 1 has been described as an example in the above-described embodiment, this is only an example and a device that executes the function provided in the movement state detection function 54 is not limited to the constituent elements included in the PET device 1. For example, the function provided in the movement state detection function 54 may be executed by a computer device such as a personal computer (PC) installed in an examination room of a hospital where the PET device 1 is installed, a medical examination room in a hospital, or the like. In this case, a display device such as a display for presenting information to the PET examination practitioner, an input interface for the PET examination practitioner to input manipulations of the computer device and information, and the like may be connected to the computer device or, for example, may be connected to the console device 40 through the network connection circuitry 44. For example, the function provided in the movement state detection function 54 may be performed by a server device on a network (not shown) or the like. In this case, it is only necessary to install at least a display device and an input interface in the examination room or the medical examination room and the server device that executes the function provided in the movement state detection function 54 may communicate with the display device or the input interface via the network (not shown). Further, only some functions provided in the movement state detection function 54 such as the division function 544 and/or the detection function 546 may be executed by the server device. In this case, the function provided in the movement state detection function 54 executed in the PET device 1 (more specifically, the console device 40) and the function provided in the movement state detection function 54 executed by the server device are performed to communicate with each other via the network (not shown) by the network connection circuitry 44, such that the overall function provided in the movement state detection function 54 may be executed. In these cases, it is only necessary for the operation and process of the function provided in the movement state detection function 54 to be equivalent to the operation and process of the function provided in the movement state detection function 54 of the above-described embodiment. Therefore, detailed description of the operation and process of the function provided in the movement state detection function 54 when executed outside the processing circuitry 50 provided in the PET device 1 is omitted.

Although the case where the movement state detection function 54 is applied to the PET device 1 has been described as an example in the above-described embodiment, this is only an example and the nuclear medicine diagnosis device to which the movement state detection function 54 is applied is not limited to the PET device. For example, the movement state detection function 54 may be applied to the PET-CT device. In this case, it is only necessary for the configuration, operation, and process of the movement state detection function 54 to be equivalent to the configuration, operation, and process of the movement state detection function 54 of the above-described embodiment. Therefore, detailed description of the configuration, operation, and processing of the movement state detection function 54 when applied to a nuclear medicine diagnosis device other than the PET device 1 is omitted.

The above-described embodiment can be represented as follows.

A nuclear medicine diagnosis device including:
processing circuitry configured to
collect radiation data obtained by detecting radiation based on radioactive medicine administrated to a subject,
divide the radiation data into a plurality of radiation data segments with predetermined time widths, and
detect a movement state of a region of interest in a body of the subject on the basis of the radiation data segments.

At least one embodiment described above includes a collector (542) configured to collect radiation data (single list mode data) obtained by detecting radiation (gamma rays) based on radioactive medicine administered to a subject (P); a divider (544) configured to divide the radiation data into a plurality of radiation data segments (list mode data segments) at predetermined time widths; and a detector (546) configured to detect a movement state of a region of interest (ROI) in a body of the subject on the basis of the radiation data segments, whereby it is possible to quantitatively detect the movement state of the region of interest in an examination (a PET examination) performed by capturing a medical image (a PET image) of the subject.

While several embodiments of the present invention have been described above, these embodiments have been presented by way of example only, and are not intended to limit the scope of the inventions. These embodiments may be embodied in a variety of other forms. Various omissions, substitutions, and combinations may be made without departing from the spirit of the inventions. The inventions described in the accompanying claims and their equivalents are intended to cover such embodiments or modifications as would fall within the scope and spirit of the inventions.

## Claims

1. A nuclear medicine diagnosis device comprising:
a collector configured to collect radiation data obtained by detecting radiation based on radioactive medicine administered to a subject;
a divider configured to divide the radiation data into a plurality of radiation data segments with predetermined time widths; and
a detector configured to detect a movement state of a region of interest in a body of the subject based on the radiation data segments.

2. The nuclear medicine diagnosis device according to claim 1, wherein the detector includes
a reconstructed image generator configured to generate reconstructed images indicating positions of the region of interest based on the radiation data segments;
a rearrangementer configured to rearrange the generated reconstructed images in order of time of respiration of the subject; and
a comparator configured to compare the positions of the region of interest between at least two rearranged reconstructed images,
wherein the movement state of the region of interest is detected based on a comparison result of performing a comparison process.

3. The nuclear medicine diagnosis device according to claim 2, wherein the rearrangementer rearranges the reconstructed images based on feature quantities of the generated reconstructed images.

4. The nuclear medicine diagnosis device according to claim 3, wherein the rearrangementer extracts the feature quantities including at least movement components in the region of interest indicated in the reconstructed images by inputting the reconstructed images to a learning network and rearranges the reconstructed images based on a degree of similarity between the feature quantities.

5. The nuclear medicine diagnosis device according to any one of claims 2 to 4, wherein the comparator compares positions of the region of interest between a first reconstructed image that is the reconstructed image indicating a maximum expiration state in the respiration of the subject and a second reconstructed image that is the reconstructed image indicating a maximum inspiration state in the respiration of the subject.

6. The nuclear medicine diagnosis device according to claim 5, wherein the comparator obtains a difference between a position of a first region of interest that is the region of interest indicated in the first reconstructed image and a position of a second region of interest that is the region of interest indicated in the second reconstructed image and outputs information indicating the obtained difference as the comparison result.

7. A region-of-interest movement state detection method comprising:
collecting, by a computer, radiation data obtained by detecting radiation based on radioactive medicine administrated to a subject;
dividing, by the computer, the radiation data into a plurality of radiation data segments with predetermined time widths; and
detecting, by the computer, a movement state of a region of interest in a body of the subject based on the radiation data segments.

8. A program for causing a computer to:
collect radiation data obtained by detecting radiation based on radioactive medicine administrated to a subject;
divide the radiation data into a plurality of radiation data segments with predetermined time widths; and
detect a movement state of a region of interest in a body of the subject based on the radiation data segments.
